# EUROPEAN PATENT APPLICATION

(11) **EP 3 159 407 A1**
(43) Date of publication of application: **26.04.2017**
(21) Application number: 15191344.9
(22) Date of filing: 23.10.2015
(51) Int. Cl.: C12N 15/11

(54) **GUIDE RNAS, METHODS AND USES**

(71) Applicant: Silence Therapeutics (London) Ltd, London W1W 8DH (GB)
(72) Inventor: The designation of the inventor has not yet been filed
(74) Representative: Stratagem IPM Limited

(57) **Abstract**

The present invention discloses modified guide RNAs which can be used with the CRISPR CAS 9 system for modifying gene expression, particularly in mammalian cells for medical or research uses.

## Description

The present invention relates to guide RNAs used in the control of gene expression by, for example, gene editing or gene disruption. In particular the guide RNAs may be target gene-specific single guide RNAs. They may be used with the Clustered Regularly Interspaced Short Palindromic repeats (CRISPR) and the CRISPR associated protein (CAS) system for editing, altering, repair of genes in mammalian cells or tissues and for research, medical or therapeutic uses. In particular the invention relates to S.pyogenese CAS9 guide RNAs amongst others.

CRISPRs are segments of prokaryotic DNA containing short repetitions of base sequences. Each repetition is followed by short segments of "spacer DNA" from previous exposures to a bacterial virus or plasmid. A set of genes was found to be associated with CRISPR repeats, and was named the CAS, or CRISPR-associated, genes. The CAS genes encode putative nuclease or helicase proteins, which are enzymes that can cut DNA. Cas9 (gene 9) is an RNA-guided DNA endonuclease enzyme associated with the CRISPR adaptive immunity system in *Streptococcus pyogenes,* among other bacteria. *S. pyogenes* utilizes Cas9 to memorize and later interrogate and cleave foreign DNA, such as invading bacteriophage DNA or plasmid DNA. Cas9 performs this interrogation by unwinding foreign DNA and checking whether it is complementary to the "spacer region" of the guide RNA. If the DNA substrate is complementary to the guide RNA, Cas9 cleaves the invading DNA. The guide RNAs can be designed to recognise a specific target gene or DNA sequence. In this sense, the CRISPR-Cas9 mechanism has a number of parallels with the RNA interference (RNAi) mechanism in eukaryotes.

The CRISPR/Cas system has been used for gene editing (adding, disrupting or changing the sequence of specific genes) and gene regulation in many species. By delivering the Cas9 protein and appropriate guide RNAs into a cell, the organism's genome can be relatively cheaply cut at any desired location. Cas9 potentially has the capability to cleave nearly any sequence complementary to the guide RNA because the target specificity of Cas9 stems from the guide RNA:DNA complementarity and not modifications to the protein itself as with like TALENs (Transcription activator-like effector nucleases) and Zinc-fingers.

While native Cas9 requires a guide RNA composed of two disparate RNAs that associate to make the guide, the CRISPR RNA (crRNA), and the trans-activating RNA (tracrRNA), Cas9 targeting has been simplified through the engineering of a chimeric single guide RNA (Jinek et al. (2012) Science 337: 816-821).

The CRISPR-Cas9 system has been adopted to induce targeted genetic changes in mammalian cells (Sternberg and Doudna, (2015) Mol Cell 58(4):568; Sampson and Weiss (2014), Bioassays 36(1):34; Zhang et al. (2014) Hum Mol Genet. 23(R1):R40). When used in mammalian cells, CRISPR-Cas9 consists of an RNA-guided nuclease (Cas9) and a short guide RNA (gRNA), which together generate site-specific breaks in the cellular DNA. These breaks are then imperfectly repaired by endogenous cellular mechanisms like non-homologous end-joining (NHEJ), creating insertions or deletions (indels) in the DNA sequence. Possible outcomes of this process are mutations at a specific site within the open reading frame of a given gene. This subsequently leads to a frame shift within the coding region, resulting in the creation of pre-mature translational STOP codons, preventing translation of the targeted specific protein.

Naturally occurring guide RNA is composed of two RNAs termed CRISPR RNA (crRNA) and trans-activating crRNA, which can be combined into a chimeric single guide RNA (sgRNA). sgRNAs are typically about 100 nucleotides (nt) long. Twenty nucleotides at the 5' end hybridize to the targeted DNA sequence by Watson-Crick base pairing and guide the Cas endonuclease to cleave the target genomic DNA. The remaining double-stranded structure at the 3' end of the sgRNA is critical for Cas9 recognition. A CRISPR-Cas9 system has been described in WO 2014/093661.

When using CRISPR for gene editing in mammalian cells or tissues, Streptococcus pyogenes Cas9 mRNA or Cas9 protein has to be introduced into the cell along with a target gene-specific single guide RNA (sgRNA) using liposomal delivery vehicles. These single-stranded RNAs like sgRNAs are rapidly degraded by intracellular RNases such as exonucleases (e.g. during the time Cas9 mRNA is translated into Cas9 protein), resulting in reduced CRISPR activity. In addition, the use of alternative delivery vehicles for delivering sgRNA in vivo such as N-acetylgalactosamine (GalNAc) will require efficient stabilization to prevent degradation by endonucleases (Sorrentino (1998) Cell Mol Life Sci.Aug;54(8):785-94).

Moreover, single-stranded RNA has been shown to activate undesirable cellular immune receptors (e.g. TLR, RIG-I) resulting in activation of cellular mechanisms that negatively affect mRNA translation (e.g. translation of co-delivered Cas9 mRNA) (Diebold (2008) Adv Drug Deliv Rev. Apr 29;60(7):813-23).

The present invention attempts to address the problems in the art by providing suitable guide RNAs.

According to the present invention there is provided a modified guide RNA for use with the CRIPR/Cas system where the guide RNA may be modified by a chemical modification of at least one nucleotide at the 2' position and/or backbone modification. The backbone modification may include modification of the thioates.

Preferably, the nucleotides that are modified are selected from a group of nucleotides which interact with the Cas amino acids in the Cas protein to effect binding of the guide RNA to Cas.

The modification may be that the 2'-OH on the nucleotide is replaced with at least one of H, OR, R, halo, SH, SR, NH2, NHR, N(R)2or CN, wherein R is C1-C6 alkyl, alkenyl or alkynyl and halo is F, Cl, Br or I.

Preferably, the modifications are 2'-O-methyl and/or 2'F.

The modified guide RNA can be applied with the S.pyogense CRISPR/Cas9 system, or any other CRISPER/Cas systems such as those in Staphylococcus aureus or Staphylococcus haemolyticus. The modification can also be made to guide RNAs for Cpf1 from Lachnospiraceae bacterium ND2006 or Cpf1 from Acidominococcus species BV3L6.

Preferred modifications and modification patters of the nucleotides of a guide RNA according to the invention are shown in SEQ ID. No. 1, 2, or 4 to 31. Other modifications and modified sequences are shown in SEQ. ID. NO. 32 to 136. Preferably the modified guide RNA sequences according to any preceding claim having the sequence and modifications in any one of

Conveniently, the modified nucleotides have at least 100%, 99%, 98%, 96%, 95%, 90 %, 85%, 80%, 75%, or 70% correspondence to a target nucleotide selected from a gene or DNA.

According to another aspect of the invention, the modified guide RNAs according to the present invention may be used in medicine. The modified guide RNAs can be used for the preparation of a medicament for the treatment of any disease, disorder or condition relating to a gene where the gene may be altering, manipulated, edited, and modified by insertion or deletion of DNA.

According to a further aspect of the invention, the modified guide RNA may be used for altering genes by deleting, substituting, repairing or inserting DNA. This can be done in animals, or microorganisms, in particular mammals and more particularly in humans. Human cells or tissue may be genetically altered or amended using the guide RNAs of the present invention and the CRISPR/Cas system known in the art in vitro and then inserted back into the patient in need thereof.

In a still further aspect of the present invention there is provided a method of altering expression of one or more genes in a cells in vitro comprising introducing into a cell a modified guide RNA according to any one of claims 1 to 5 in a CRISPR-Cas system, wherein the guide RNA targets the gene and the Cas protein cleaves the genomic loci of the DNA molecules encoding the one or more gene products, whereby expression of the one or more gene products is altered

Preferably, the CRISPR-Cas system comprising a Cas protein and one or more modified guide RNAs according to the invention comprises where the guide RNA corresponds to a target a gene.

In another aspect of the invention there is provided a vector comprising a CRISPR-Cas system comprising a Cas protein and one or more modified guide RNAs according to the invention comprises where the guide RNA corresponds to a target a gene.

In a further aspect of the invention there is provided a cell comprising a CRISPR-Cas system or a vector comprising a Cas protein and one or more modified guide RNAs according to the invention comprises where the guide RNA corresponds to a target a gene

In another aspect of the invention there is provided a pharmaceutical composition comprising a modified guide RNA according and a CRISPR-Cas system and a pharmaceutically acceptable carrier or excipient. The pharmaceutical composition may include a vector or a cell with the modified guide RNA of the invention.

In a still further aspect of the invention there is provided a composition comprising a modified guide RNA and at least one delivery means selected from GalNAC, polymers, liposomes, peptides, aptameres, antibodies, viral vectors, folate or transferrin.

The modified guide RNAs may be single guide RNAs.

The guide RNAs may be synthetic or chemically modifies guide RNAs. The nucleotides in the guide RNA that are modified may be those corresponding to one or more nucleotides in the binding region of the guide RNA with Cas9 and/or the nucleotides in the binding region of the guide RNA with the target DNA. Remaining unmodified nucleotides of the guide RNA are those required to be identified for minimal binding of Cas9 to the 2'-OH location on the bases.

The CAS protein may be selected from Cas protein type II CRISPR enzyme system or Cas9 protein of S.pyogenes. Any mutated Cas9 protein derived from other organisms are included.

The nucleotides are modified at the 2' position of the sugar moiety of the nucleotide. Preferably, the 2'-OH group of the sugar moiety is replaced by a group selected from H, OR, R, halo, SH, SR, NH2, NHR, N(R)2or CN, wherein R is C1-C6 alkyl, alkenyl or alkynyl and halo is F, Cl, Br or I.

Other modifications may include, inverted (deoxy) abasics, amino, fluoro, chloro, bromo, CN, CF, methoxy, imidazole, caboxylate, thioate, C1 to C10 lower alkyl, substituted lower alkyl, alkaryl or aralkyl, OCF3, OCN, O-, S-, or N-alkyl; O-, S-, or N-alkenyl; SOCH3; SO2CH3; ONO2; NO2, N3; heterozycloalkyl; heterozycloalkaryl; aminoalkylamino; polyalkylamino or substituted silyl.

In one embodiment the 2'position modification is 2'-O-methly modification.

In an alternative embodiment the 2' modification is a 2'F modification, or a combination of modifications selected from the group 2'-O-methyl or 2'F.

In a further embodiment, all the modifications are 2'-O-methyl.

Synthetic RNA production yield is based on sequences and modifications. 2'-O-methyl modifications have been shown to increase coupling efficacy during RNA synthesis and therefore increase yield of chemically synthesized RNA.

Furthermore, nucleotides may be modified by phosphorothioates. Phosphothioates (PS) bond substitutes a sulphur atom for a non-bridging oxygen in the phosphate backbone of an oligonucleotide.

Exemplary nucleotides of the invention include, but are not limited to, ribonucleic acids (RNAs), deoxyribonucleic acids (DNAs), threose nucleic acids (TNAs), glycol nucleic acids (GNAs), peptide nucleic acids (PNAs), locked nucleic acids (LNAs, including LNA having a β- D-ribo configuration, a-LNA having an a-L-ribo configuration (a diastereomer of LNA), 2'-amino-LNA having a 2'-amino functionalization, and 2'-amino- a-LNA having a 2'-amino functionalization) or hybrids thereof.

Any CRISPR/ CAS system can use the modified guide RNAs of the present invention.

The guide RNAs may hybridise with target sequences in the genomic loci of DNA molecules encoding one or more gene products.

The guide RNA may comprise a guide sequence fused to a tracr sequence.

Two or more modified guides according to the present invention may be used in the same CRISPR/CAS system and so can knock out multiple genes in the same genome.

The guide RNA sequences are usually up to a 100 nucleotides long. They are in a single stranded structure, or double stranded and can have hairpin loops. The guide RNA is a combination of the endogenous bacterial crRNA and tracrRNA into a single chimeric guide RNA (sgRNA) transcript. The sgRNA combines the targeting specificity of the crRNA with the scaffolding properties of the tracrRNA into a single transcript. The crRNA region is a 20-nucleotide sequence that can be designed to be homologous to a region in a gene of interest and will direct Cas9 nuclease activity. The 3' end of the DNA target sequence must have a proto-spacer adjacent motif (PAM) sequence (5'-NGG-3'). The 20 nucleotides upstream of the PAM sequence can be selected as targeting sequence (crRNA) and Cas9 nuclease will cleave approximately 3 bases upstream of the PAM. Crystal structures of sgRNA/DNA-bound Cas9 show that the spacer (guide) and stem-loop motifs at the 5' end of the sgRNA primarily contact the α-helical lobe where as two hairpins at the 3' end bind the outside face of the nuclease lobe.

Analysis of Cas9 crystal structure also revealed detailed interactions between Cas9 and sgRNA scaffold. Amino acids of Cas9 interact with the sgRNA backbone phosphate groups, 2'-hydroxyl groups and nucleobases via hydrogen bonds, hydrophobic interactions and van der Waals contacts and aromatic stacking interactions.

Jiang et al. (2015) identified additional bases that bind Cas9 via their 2'-hydroxyl groups, suggesting that not all 2'-hydroxyl group interactions are necessary for Cas9:sgRNA interaction.

RNAs can be stabilized by modification of their 2'-hydroxy groups (e.g. 2'-O-methyl- or 2'-fluoro-modifications) or at the backbone phosphate group (e.g. phosphorothioate modification).

Hendel et al. (2015) has described the use of chemically modified guide RNAs for Streptococcus pyogenes. Chemical modifications comprising 2'-O-methyl (M), 2'-O-methyl 3'phosphorothioate (MS), or 2'-O-methyl 3'thioPACE (MSP) were incorporated at the three terminal nucleotides at both the 5' and 3' ends. Modifications were introduced into the first and last three nucleotides of the guide RNA only. These modifications provide protection from degradation by cellular exonucleases. However, as the bulk of internal nucleotides of the sgRNA is unmodified, the sgRNA is still sensitive to degradation by endonucleases.

Modification of internal nucleotides can lead to non-functional guide RNAs (e.g. modification of nucleotides that are required for Cas9 binding).

Modified sgRNAs may be delivered to cells and tissues using viral, polymeric and liposomal formulations. Further delivery vehicles may be cell-penetrating peptides, aptameres, conjugates (e.g. folate, transferrin, N-acetylgalactosamine (GalNAc) and antibody approaches.

Cas9 may be delivered in the form of Cas9 encoding DNA, Cas9 encoding mRNA or as Cas9 protein using viral, polymeric and liposomal formulations. Further delivery vehicles may be cell-penetrating peptides, aptameres, conjugates (e.g. folate, transferrin, N-acetylgalactosamine (GalNAc) and antibody approaches.

The present inventors have surprisingly found that distinct additional internal modifications of the Guide RNA prevents degradation by exo- and endonucleases and at the same time allows Cas9 binding.

The inventors show for the first time that modification of *Streptococcus pyogenes* specific sgRNA at distinct 2'-hydroxy positions by 2'-O-methyl or 2'-fluoro modifications increases sgRNA integrity, secondary structure stability and reduces immune stimulatory activity of sgRNAs resulting in increased CRISPR activity.

Importantly, modifications of the invention can be applied to any guide RNAs, independent of the specific target gene sequence.

The target gene can relate to any organism including plants, fungi, prokaryotes, animals. The invention can be used for gene modification of any mammals including, humans.

### Details of the invention:-

The inventors for the first time describe a novel and universally applicable, i.e. target independent modification pattern for synthetic chimeric guide RNA for Streptococcus pyogenes Cas9. Guide RNAs may be modified using 2'-O-methyl-, 2'-fluoro and any other modifications located at the 2' position. The modified guide RNA may be used for in vivo and ex vivo gene editing/gene disruption applications in combination with Streptococcus pyogenes Cas9 protein, Streptococcus pyogenes Cas9 mRNA or Streptococcus pyogenes Cas9 encoding DNA plasmids. Gene disruption applications aim at disease-relevant genes and gene editing applications aim at mutated disease-relevant genes. Indications involve autosomal dominant and recessive mutations.

The selection of modified sgRNA nucleotides was based on whether or not Cas9 amino acids bind these bases at their 2'-hydroxyl group.

In one embodiment, the Streptococcus pyogenes Cas9 mRNA modified guide RNAs may consist of a fully modified 96 nucleotides long guide RNA except for the following bases which remain unmodified at their 2'-hydroxl moieties:
N-6, N-5, N-2, U+2, U+3, U+4, G+23, U+24, U+25, A+27, U+45

Here, the "Guide-1" modification pattern is based on the following sequence (SEQ ID NO. 1): 
5’-
NNNNNNNNNNNNNNNNNNNNGGUUUUAGAGCUAGAAAUAGCAAGUUAAAAUAA
GGCUAGUCCGUUAUCAACUUGAAAAAGUGGCACCGAGUCGGUGC - 3′
 N = target specific sequence; X = ribonucleotides with 2'-hydroxyl group; X = ribonucleotides with 2'-O-methyl group, 2'-flouro group or any other modification; * = backbone phosphorothioate modification

In a second embodiment, the Guide-2 modification pattern (**SEQ ID NO**.**2**) has the follwoing sequence:-

5‘ -
NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC -3‘

Further emobiments and nuclotide modification sequences are provided below:-
Guide 17 modification pattern (**SEQ ID NO.8**) 
5‘-
NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC -3‘
Guide-15 modification pattern (**SEQ ID NO.9**) 
5‘-
NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC-3‘
Guide-37 modification pattern (**SEQ ID NO.10**) 
5'-
NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC-3'
Guide-39 modification pattern (**SEQ ID NO.11**) 
5‘-
NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC-3‘
Guide-41 modification pattern (**SEQ ID NO.12**) 
5‘-
NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC-3‘
Guide-43 modification pattern (**SEQ ID NO.13**) 
5‘-
NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC-3‘
Guide-42 modification pattern (**SEQ ID NO.14**)
Guide-38 modification pattern (**SEQ ID NO.15**) 
5‘-
NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC-3‘
Guide-50 modification pattern (**SEQ ID NO.16**) 
5‘-
NNNNNNNNNNNNNNNNN*N*NNN*NGUUUUAGAGCUAGAAAUAGCAAGUUAAAA
UAAGGCUAGUCCGUUAUCAACUUGAAAAAGUGGCACCGAGUCGGUGC-3‘
Guide-51 modification pattern (**SEQ ID NO.17**) 
5'-
NNNNNNNNNNNNNNNNNNNNGU*U*U*UAGAGCUAGAAAUAGCAAGUUAAAAU
AAGGCUAGUCCGUUAUCAACUUGAAAAAGUGGCACCGAGUCGGUGC-3'
Guide-52 modification pattern (**SEQ ID NO.18**) 
5‘-
NNNNNNNNNNNNNNNNNNNNGUUUUAGAGCUAGAAAUAGCAAG*U*U*AA*AA
UAAGGCUAGUCCGUUAUCAACUUGAAAAAGUGGCACCGAGUCGGUGC-3‘
Guide-53 modification pattern (**SEQ ID NO.19**) 
5‘-
NNNNNNNNNNNNNNNNNNNNGUUUUAGAGCUAGAAAUAGCAAGUUAAAAUAA
GGCUAG*UCCG*U*U*A*UCA*ACUUGAAAAAGUGGCACCGAGUCGGUGC-3‘
Guide-54 modification pattern (**SEQ ID NO.20**) 
5'-
N*NNNNNNNNNNNNNNN*N*NNN*NG*U*U*U*UAGAGCUAGAAAUAGCAA*G*U*
U*A*A*AAUAAGGCUAG*UCC*G*U*U*A*UC*A*ACUUGAAAAAGUGGCACCGAG
UCGGUGC-3'
Guide-55 modification pattern (**SEQ ID NO.21**) 
5'-
N*NNNNNNNNNNNNNNNNNNNGUU*U*UAGAGCUAGAAAUAGCAA*G*U*U*A*
A*AAUAAGGCUAG*UCC*G*U*U*A*UC*A*ACUUGAAAAAGUGGCACCGAGUCG
GUGC-3'
Guide-56 modification pattern (**SEQ ID NO.22**) 
5'-
NNNNNNNNNNNNNNNNNNNNGU*U*U*UAGAGCUAGAAAUAGCAAG*U*U*AAA
AUAAGGCUAGUCCG*U*U*A*UCAACUUGAAAAAGUGGCACCGAGUCGGUGC-3'
Guide-63 modification pattern (**SEQ ID NO.23**) 
5‘-
NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC-3‘
Guide-67 modification pattern (**SEQ ID NO.24**) 
5‘-
NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC-3‘
Guide-68 modification pattern (**SEQ ID NO.25**) 
5‘-
N*N*N*NNNNNNNNNNNNNNNNNGUUUUAGAGCUAGAAAUAGCAAGUUAAAAU
AAGGCUAGUCCGUUAUCAACUUGAAAAAGUGGCACCGAGUCGG*U*G*C-3‘
Guide-69 modification pattern (**SEQ ID NO.26**) 
5‘-
N*N*N*NNNNNNNNNNNNNNNNNGUUUUAGAGCUAGAAAUAGCAAGUUAAAAU
AAGGCUAGUCCGUUAUCAACUUGAAAAAGUGGCACCGAGUCGG*U*G*C-3‘
Guide-70 modification pattern (**SEQ ID NO.27**)

5‘-
NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC-3‘

Other Guide RNA modification patters are:-

| | | |
|---|---|---|
| SEQ ID NO. 28 | Guide-22 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 29 | Guide-23 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 30 | Guide-24 | NNNNNNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 31 | Guide-25 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |

Guide RNAs of other microorganisms can be similarly modified, for example, those of Staphylococcus aureus and Staphylococcus haemolyticus. Guide RNAs for Cpf1 from Lachnospiraceae bacterium ND2006 and Acidaminococcus species BV3L6 can be modified according to the present invention.

The guide RNAs according to the present invention can be used for preventing or treating any gene related condition.

The modified guide RNAs of the present invention can be used with the CRISPR system to knocking out genes, amplifying genes and repairing particular mutations associated with DNA repeat instability and neurological disorders, correcting defects associated with a wide range of genetic diseases. As will be apparent to a person skilled in the art, it is envisaged that the modified guide RNAs of the present invention can be used with the CRISPR system to target any polynucleotide sequence of interest.

Preferably, the modified guide RNAs can be used in the CRISPR system to target genes in mammalian cells, organs, organisms. In particular the guide RNAs of the present invention can be used to target genes related to a disease. The types of diseases or medical conditions and the associated genes with such diseases or medical conditions can be the gene targets for the CRISPR system using the modified guide RNAs of the present invention. Examples of such diseases or medical conditions include but are not limited to:-
liver diseases, Hepatitis B virus, Alzheimer's disease, schizophrenia disorders, ALS, inflammation and immune related diseases and disorders, Parkinson's Disease, blood and coagulation diseases and disorders, cell dysregulation and oncology diseases and disorders, neoplasia, age-related macular degeneration, Trinucleotide Repeat Disorders, Fragile X Syndrome, secretase related disorders, prion-related disorders, drug addiction, autism, Inflammation, metabolic diseases, kidney diseases, diseases and disorders due to a protein malfunction, muscular dystrophy, muscular/skeletal diseases and disorders, multiple sclerosis, neurological and neuronal diseases and disorders, occular diseases and disorders, Epilepsy, AIDS or Alpha 1-Antitrypsin Deficiency.

Genes associated with cellular functions and cell signalling can also be targeted. For example genes involved in various cellular pathway signalling, hormone signalling, metabolic functions, biosynthetic pathways, or receptor activation or de-activation such as PI3K/AKT, ERK/MAPK, PTEN, p53, SAPK/JNK, IGF-1, IL-6, IL-2, IL-4, PPAr/RXR, NF-KB, Wnt & Beta catenin, PPAR, PDGF, VEGF, FGF, GM-CSF, JAK/Stat, IL-10, VDR/RXR, p38 MAPK, neurotrophin/TRK, FXR/RXR, LPS/IL-1 glucocorticoid receptor, Cytochrome p450, axonal guidance, ephrin receptor, actin cytoskeleton, Huntington's Disease, Parkinson's disease, apoptosis, B-Cell receptor, leukocyte and integrin signaling, acute phase responses, aryl hydrocarbon receptor xenobiotic metabolism, interferon, neuregulin, insulin receptor, hepatic cholestasis, NRF2-mediated oxidative stress, fibrosis, hepatic stellate cell activation, Fc Epsilon RI, G-Protein coupled receptor, glutamate receptor, pentose and glucoronate, inositol phosphate metabolism, natural killer cell signaling, cell cycle: G1/S checkpoint regulation, T Cell receptor, death receptor signaling, nicotinate and nicotinamide metabolism, chemokine, signaling, any synaptic signalling, estrogen receptor, protein ubiquitination pathway, TGF-beta signaling, eicosanoid, Toll-like receptor, calcium signaling, EGF, hypoxia, LXR/RXR function, amyloid processing, cell cycle signalling pathways, nitric oxide and other pathways in the cardiovascular system, mitochondrial function, developmental genes and neurological genes, cAMP-mediated signaling, mitochondrial dysfunction notch signaling, endoplasmic reticulum stress pathway, antigen presentation pathway, NRF2-mediated oxidative stress response, pentose phosphate pathway coagulation system dopamine receptor, Cardiac & Beta Adrenergic signalling, glycolysis and gluco-neogenesis, sonic hedgehog, circadian rhythm, glycerophospholipid and phospholipid degradation.

Metabolic and biosynthetic pathways include but are not limited to any amino acid metabolism, purine, tryptophan, lysine degradation, nucleotide excision repair pathway, starch and sucrose, aminosugars, arachidonic acid, butanoate, glutathione, fatty acid, glycerophospholipid, histidine glycerolipid, linoleic acid, methionine, pyruvate, arginine and proline, pyrimidine, any sugar metabolism, coumarine and lignin biosynthesis, biosynthesis of steroids, bile acid biosynthesis citrate cycle, inositol, xenobiotics, methane, phenylalanine, propanoate, sphingolipid, aminophosphonate, androgen, estrogen, ascorbate, aldarate, cysteine, retinol, riboflavin, tyrosine, ubiquinone, valine, leucine, isoleucine, glycine, serine, threonine and lysine.

The modified guide RNAs in a CRISPR CAS system may be formulated using any suitalbe system or vehicle which woudl be well within teh knowledge of a person skilled in the art. Pharmacutical compositions of the invetions can include any pharmaceutically acceptable carrier or vehicle known in the. The pharmaceutical compositions may include a combination of other therapeutics.

Appropriate dosages using the modfied guide RNAs of the present invention that are effective for threapeutic or prophalactic applicaitons may be used.

Formulations of the guide RNAs can include one or more excipients such as solvents, dispersion media, diluents, or other liquid vehicles, dispersion or suspension aids, surface active agents, isotonic agents, thickening or emulsifying agents, preservatives, lipidoids, liposomes, lipid nanoparticles, polymers, lipoplexes, core-shell nanoparticles, peptides, proteins, cells transfected with polynucleotide, primary construct, or mmRNA (e.g., for transplantation into a subject), hyaluronidase, nanoparticle mimics and combinations thereof.

Various excipients for formulating pharmaceutical compositions and techniques for preparing the composition are known in the art. The use of a conventional excipient medium may be contemplated within the scope of the present disclosure, except insofar as any conventional excipient medium may be incompatible with a substance or its derivatives, such as by producing any undesirable biological effect or otherwise interacting in a deleterious manner with any other component(s) of the pharmaceutical composition. Formulations of the pharmaceutical compositions described herein may be prepared by any method known to a person skilled in the art.

A pharmaceutical composition in accordance with the present invnetion may be prepared, packaged, and/or sold in bulk, as a single unit dose, and/or as a plurality of single unit doses. As used herein, a "unit dose" refers to a discrete amount of the pharmaceutical composition comprising a predetermined amount of the active ingredient. The amount of the active ingredient may generally be equal to the dosage of the active ingredient which would be administered to a subject and/or a convenient fraction of such a dosage including, but not limited to, one-half or one-third of such a dosage. For example, the composition may comprise between 0.1% and 99% (w/w) of the active ingredient.

In some embodiments, the formulations may contain at least one modified guide RNA. In other formulations two or more modified guide RNAs may be used to target multiple genes. In some embodiments, the particle size of the lipid nanoparticle may be increased and/or decreased. The change in particle size may be able to help counter biological reaction such as, but not limited to, inflammation or may increase the biological effect of the modified mRNA delivered to mammals.

Pharmaceutically acceptable excipients used in the manufacture of pharmaceutical compositions include, but are not limited to, inert diluents, surface active agents and/or emulsifiers, preservatives, buffering agents, lubricating agents, and/or oils. Such excipients may optionally be included in the pharmaceutical formulations of the invention.

Liposomes may includes those formed from 1,2-dioleyloxy-N,N- dimethylaminopropane (DODMA) liposomes, DiLa2 liposomes from Marina Biotech (Bothell, WA), 1,2-dilinoleyloxy-3-dimethylaminopropane (DLin-DMA), 2,2-dilinoleyl- 4-(2-dimethylaminoethyl)-[1,3]-dioxolane (DLin-KC2-DMA), and liposomes which may deliver small molecule drugs such as, but not limited to, DOXIL® (from Janssen Biotech, Inc. (Horsham, PA).

In one embodiment, the polynucleotides, primary constructs and/or mmRNA may be formulated in a lipid vesicle which may have crosslinks between functionalized lipid bilayers, or lipid-polycation complex. The liposome formulation may be influenced by, but not limited to, the selection of the cationic lipid component, the degree of cationic lipid saturation, the nature of the PEGylation, ratio of all components and biophysical parameters such as size, or polycationic composition.

In one embodiment, pharmaceutical compositions described herein may include, without limitation, liposomes such as those formed from the synthesis of stabilized plasmid-lipid particles (SPLP) or stabilized nucleic acid lipid particle (SNALP) that have been previously described and shown to be suitable for oligonucleotide delivery in vitro and in vivo.

In another embodiment, the lipid nanoparticles may be engineered to alter the surface properties of particles so the lipid nanoparticles may penetrate the mucosal barrier. Mucus is located on mucosal tissue such as, but not limited to, oral (e.g., the buccal and esophageal membranes and tonsil tissue), ophthalmic, gastrointestinal (e.g., stomach, small intestine, large intestine, colon, rectum), nasal, respiratory (e.g., nasal, pharyngeal, tracheal and bronchial membranes), genital (e.g., vaginal, cervical and urethral membranes).

The formulations may use nanoparticles larger than 10-200 nm which are preferred for higher drug encapsulation efficiency and the ability to provide the sustained delivery of a wide array of drugs have been thought to be too large to rapidly diffuse through mucosal barriers. fluorescence recovery after photobleaching (FRAP) and high resolution multiple particle tracking (MPT).

The formulations can be made for controlled release and/or targeted delivery.

The lipid nanoparticle engineered to penetrate mucus may include surface altering agents such as, but not limited to, mmRNA, anionic protein (e.g., bovine serum albumin), surfactants (e.g., cationic surfactants such as for example dimethyldioctadecyl- ammonium bromide), sugars or sugar derivatives (e.g., cyclodextrin), nucleic acids, polymers (e.g., heparin, polyethylene glycol and poloxamer), mucolytic agents (e.g., N- acetylcysteine, mugwort, bromelain, papain, clerodendrum, acetylcysteine, bromhexine, carbocisteine, eprazinone, mesna, ambroxol, sobrerol, domiodol, letosteine, stepronin, tiopronin, gelsolin, thymosin β4 dornase alfa, neltenexine, erdosteine) and various DNases including rhDNase. The surface altering agent may be embedded or enmeshed in the particle's surface or disposed (e.g., by coating, adsorption, covalent linkage, or other process) on the surface of the lipid nanoparticle.

In a furhter embodiment, guide RNA of the present invention and the CRISPR system may be formulated as a lipoplex, such as, without limitation, the ATUPLEX™ system, the DACC system, the DBTC system and other siRNA-lipoplex technology.

The liposomes, lipoplexes, or lipid nanoparticles may be used to improve the efficacy of the modified guide RNAs for example by increasing cell transfection, increase the translation of encoded protein or increase the stability. A cell penetrating peptide may be used with the pharmaceutical formulations of the present invention such as a cell-penetrating peptide sequence attached to polycations that facilitates delivery to the intracellular space, e.g., HIV-derived TAT peptide, penetratins, transportans, or hCT derived cell-penetrating peptides

Alternatively, the lipid nanoparticle may be encapsulated into any polymer or hydrogel known in the art which may form a gel when injected into a subject. As another non-limiting example, the lipid nanoparticle may be encapsulated into a polymer matrix which may be biodegradable.

In yet anotehr embodiment, the pharmaceutical compositions may be sustained release formulations. In a further embodiment, the sustained release formulations may be for subcutaneous delivery. Sustained release formulations may include, but are not limited to, PLGA microspheres, ethylene vinyl acetate (EVAc), poloxamer, GELSITE® (Nanotherapeutics, Inc. Alachua, FL), HYLENEX® (Halozyme Therapeutics, San Diego CA), surgical sealants such as fibrinogen polymers (Ethicon Inc. Cornelia, GA), TISSELL® (Baxter International, Inc Deerfield, IL), PEG-based sealants, and COSEAL® (Baxter International, Inc Deerfield, IL).

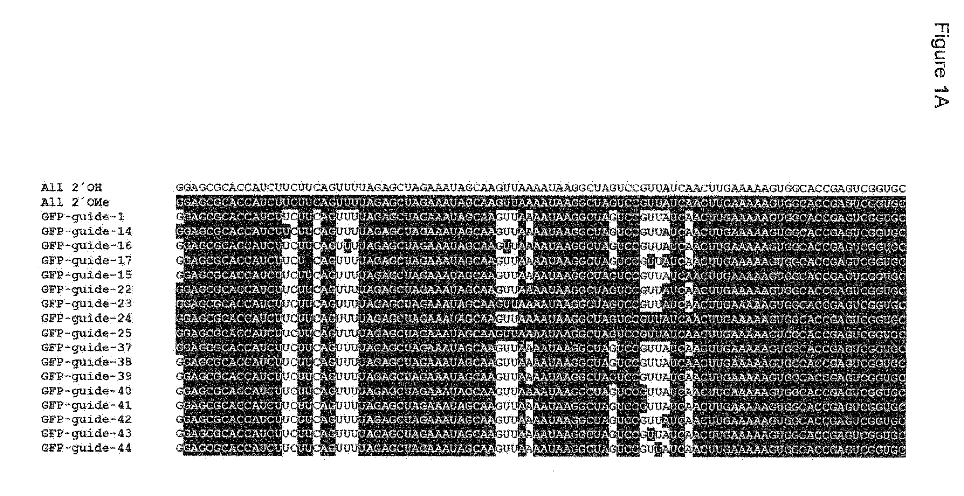

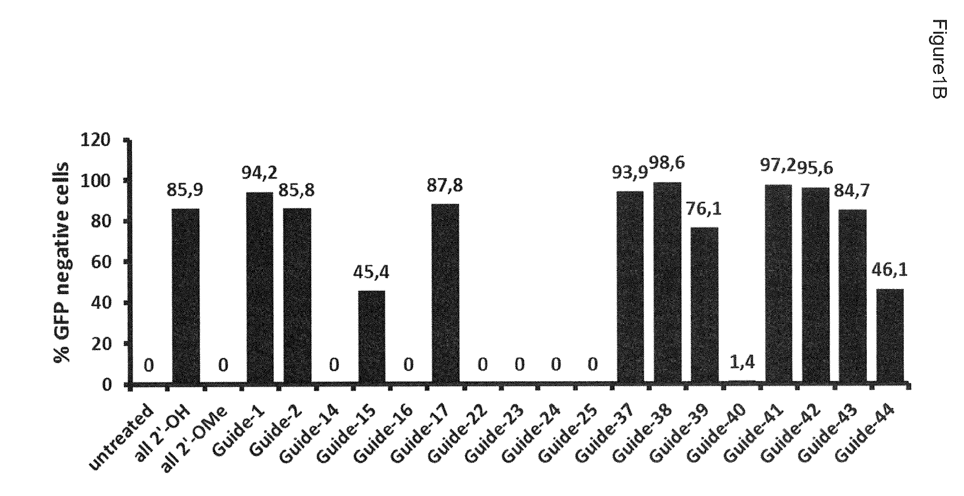

The invention will now be described in more detail with reference to the following figures and examples for illustration purposes only, in which:-
**Figure 1** shows the various modified Guide RNAs tested and their activity in GFP Hela cells: **(A)** Sequences (5'-3') of guide RNAs tested. Italic N = target specific sequence; X = ribonucleotides with 2'-hydroxyl group; X = ribonucleotides with 2'-O-methyl group. **(B)** Analysis of CRISPR activity using modified guide RNAs listed in Figure 1A shown by flow cytometry analysis of reduced GFP expression in GFP-Hela cells 4 days after transfection of Cas9 mRNA with variants of modified GFP-specific guide RNA. Modified Guide 1, 2, 17, 37, 38, 39, 41, 42 and 43 are equally active as nonmodified Guide RNA at saturating concentration (0.1ug/ml),

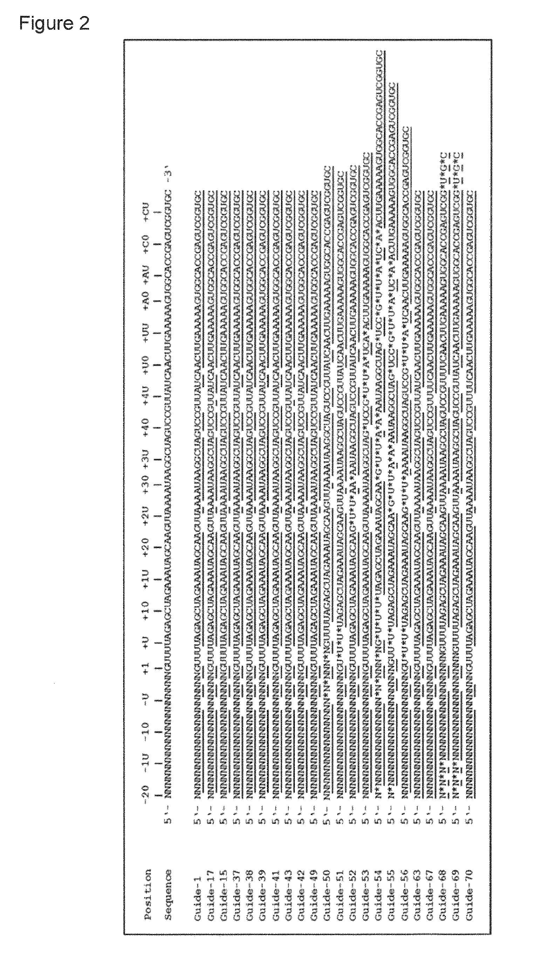

**Figure 2** shows the 2'- modification patters adopted in the Guides, for example, Guide-1, -2, -17, -15, -37, -38, -39, -41, -43, -42, -49, -50, -51, -52, -53, -54, -55, -56, -57, -58, -59 and - 70; N = target specific sequence; X = ribonucleotides with 2'-hydroxyl group; X = ribonucleotides with modifications at the 2' position of the sugar, wherein the 2'-OH group of said sugar-modified ribonucleotide is replaced by a group selected from H, OR, R, halo, SH, SR, NH2, NHR, N(R)2or CN, wherein R is C1-C6 alkyl, alkenyl or alkynyl and halo is F, Cl, Br or I; * = backbone phosphorothioate modification

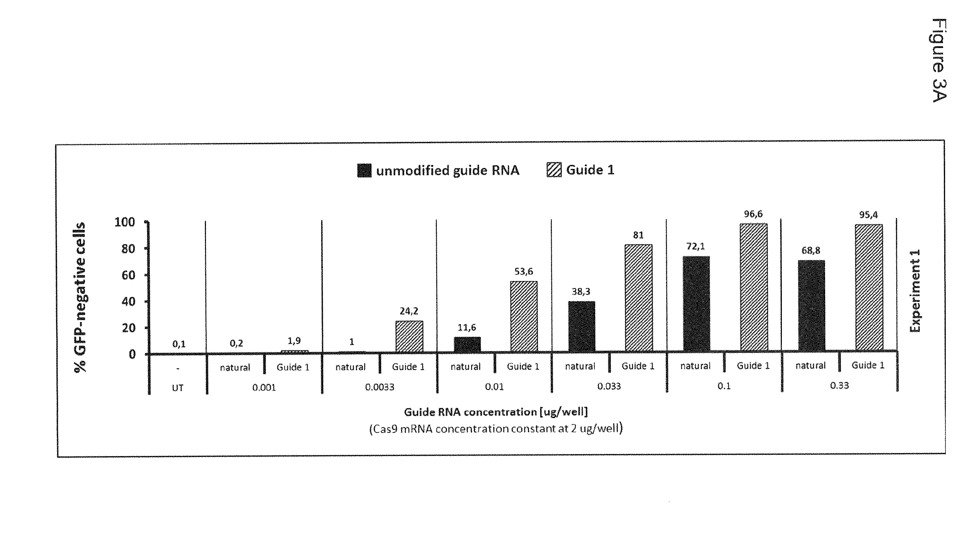

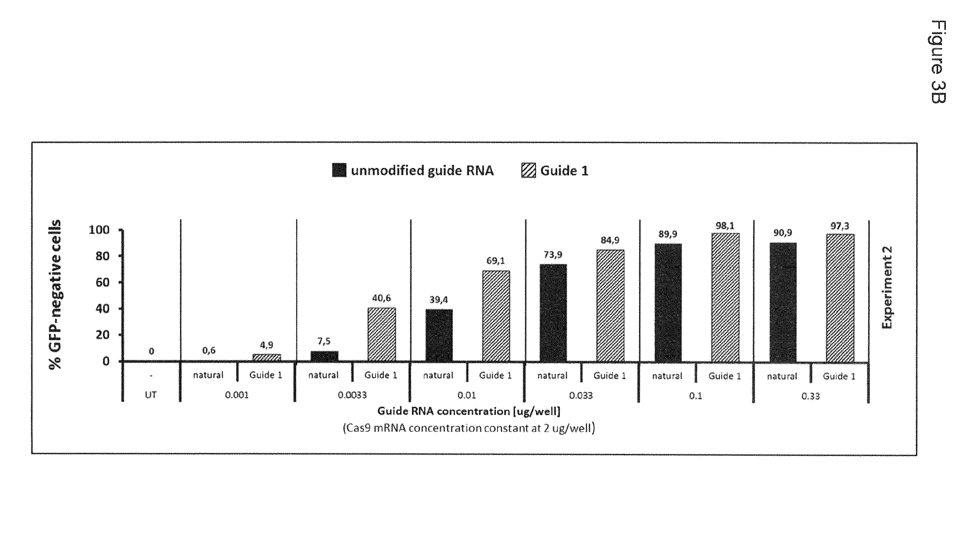

**Figure 3A** **and** **3B** show titration of Guide1 and natural Guide demonstrating improved activity of Guide1. Flow cytometry analysis from two independent experiments of GFP-Hela cells 4 days after transfection of Cas9 mRNA with indicated concentrations of natural guide RNA or Guide-1-modified guide RNA.

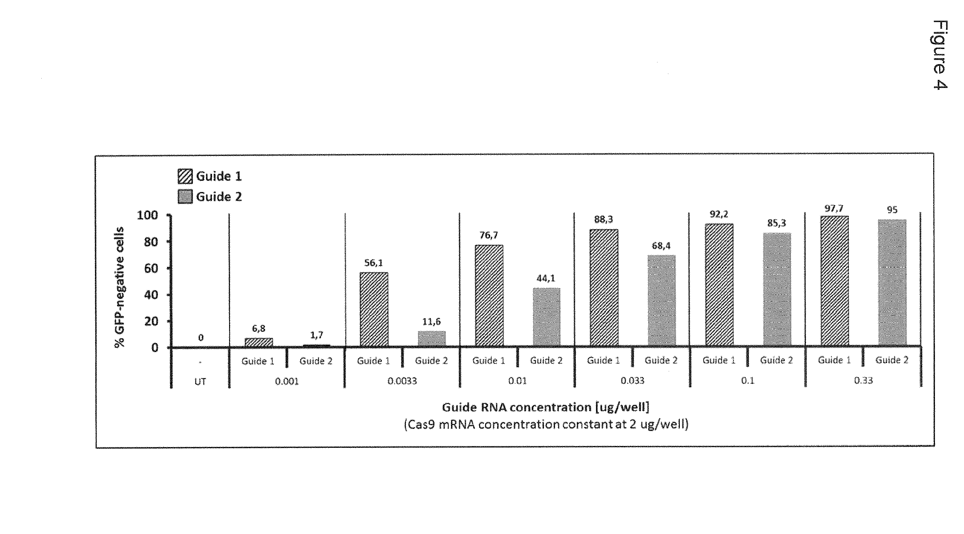

**Figure 4** shows titration of Guide1 and Guide 2, demonstrating improved activity of Guide1. Flow cytometry analysis of GFP-Hela cells 4 days after transfection of Cas9 mRNA with guide RNA version Guide-1 or Guide-2.

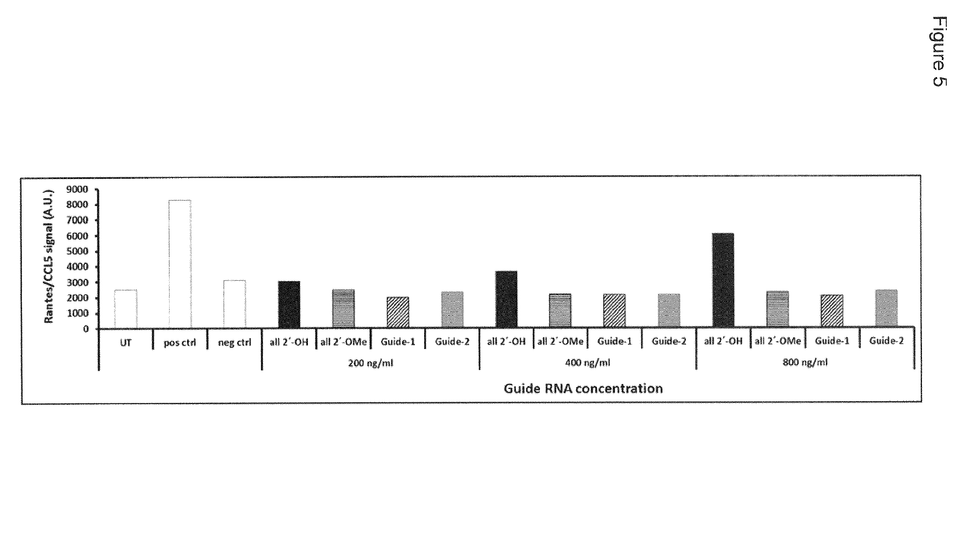

**Figure 5** shows an absence of immune stimulation of modified Guide RNAs. Flow cytometry analysis of Rantes/CCL5 release from human PBMC cells 24 hours after transfection natural guide RNA (all 2'-OH), fully 2'-O-methylated guide RNA (all 2'-OMe) and with partially 2'-O-methylated guide RNA version Guide-1 or Guide-2.

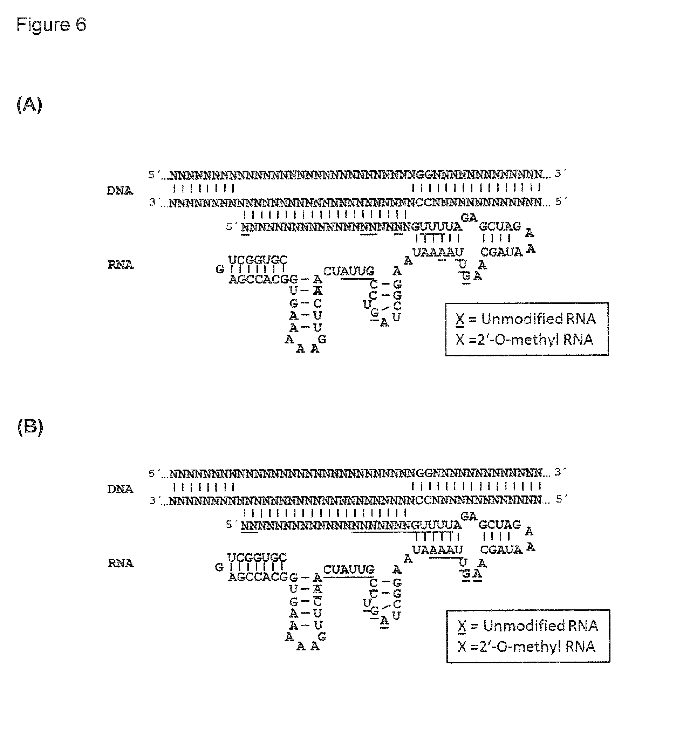

**Figure 6** shows the modifications made to the natural guide RNA and referred. (A) is Guide - 1 (**SEQ ID NO. 1**). The one that are underlined are unmodified nucleotides.(B) is Guide-2 (**SEQ ID NO. 2**) - The nucleotides are modified at the 2' position of the sugar. wherein the 2'-OH group of said sugar-modified ribonucleotide is replaced by a group selected from H, OR, R, halo, SH, SR, NH2, NHR, N(R)2or CN, wherein R is C1-C6 alkyl, alkenyl or alkynyl and halo is F, Cl, Br or I.

### Examples

### Example 1 - Preparation of modified Guides

The GFP-specific guide RNA sequence (20nt) and sequence of single chimeric guide RNA specific for Streptococcus pyogenes Cas9 has been described (Mali et al., Science. 2013 February 15; 339(6121): 823-826 and Jinek et al., Science. 2012 Aug 17;337(6096):816-21, respectively) and the methods are incorporated herein by reference.

GFP-specific chimeric guide RNAs were synthesized at Biospring GmbH (Germany). Human-codon-optimized version of the Streptococcus pyogenes Cas9 mRNA was purchased from TriLink Biotechnologies (USA).

### Example 2 - Transfection of HeLa cells

HeLa cells stably expressing the green fluorescent protein (GFP) were seeded on 6-well plates (250,000 cells/well) 24h before transfection.

Cells are co-transfected with Cas9 mRNA and differently modified guide RNAs targeting the GFP gene.

Transfections were carried out using Lipofectamine MessengerMax (Life technologies, USA) according to the manufacturer's protocol. After 24h, cells were trypsinized and transferred to 100 mm cell culture dishes. After 96h cells were trypsinized, washed twice with PBS and analysed for GFP fluorescence using flow cytometry (LSRFortessa, BD Bioscences, USA).

Results are presented in **Figures 1 - 4**.

**Table 1 - shows the sequence of modified guide RNAs in 5'-3' orientation: N = target specific sequence (for GFP: GGAGCGCACCATCTTCTTCA); X = ribonucleotides with 2'-hydroxyl group; X = ribonucleotides with 2'-O-methyl group, 2'-flouro group or any other modification; * = backbone phosphorothioate modification**

| | | |
|---|---|---|
| SEQ ID NO. 3 | All 2'-OH | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 4 | All 2'-OMe | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 1 | Guide-1 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 2 | Guide-2 | NNNNNNNNNNNNNNNNNNNNGUUUUAGAGCUAG AAAUAGCAAGUUAAAAUAAGGCUAGUCCGUUAU |
| | | CAACUUGAAAAAGUGGCACCGAGUCGGUGC |
| SEQ ID NO. 8 | Guide 17 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 9 | Guide-15 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 10 | Guide-37 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 11 | Guide-39 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 12 | Guide-41 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 13 | Guide-43 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 14 | Guide-42 | NNNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 15 | Guide-38 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 16 | Guide-50 | NNNNNNNNNNNNNNNNN*N*NNN*NGUUUUAGAGC UAGAAAUAGCAAGUUAAAAUAAGGCUAGUCCGU UAUCAACUUGAAAAAGUGGCACCGAGUCGGUGC |
| SEQ ID NO. 17 | Guide-51 | NNNNNNNNNNNNNNNNNNNNGU*U*U*UAGAGCU AGAAAUAGCAAGUUAAAAUAAGGCUAGUCCGUU AUCAACUUGAAAAAGUGGCACCGAGUCGGUGC |
| SEQ ID NO. 18 | Guide-52 | NNNNNNNNNNNNNNNNNNNNGUUUUAGAGCUAG AAAUAGCAAG*U*U*AA*AAUAAGGCUAGUCCGU UAUCAACUUGAAAAAGUGGCACCGAGUCGGUGC |
| SEQ ID NO. 19 | Guide-53 | NNNNNNNNNNNNNNNNNNNNNNGUUUUAGAGCUAG AAAUAGCAAGUUAAAAUAAGGCUAG*UCCG*U*U *A*UCA*ACUUGAAAAAGUGGCACCGAGUCGGUG C |
| SEQ ID NO. 20 | Guide-54 | N*NNNNNNNNNNNNNNN*N*NNN*NG*U*U*U*UAG AGCUAGAAAUAGCAA*G*U*U*A*A*AAUAAGGCU AG*UCC*G*U*U*A*UC*A*ACUUGAAAAAGUGGC ACCGAGUCGGUGC |
| SEQ ID NO. 21 | Guide-55 | N*NNNNNNNNNNNNNNNNNNNNGUU*U*UAGAGCU AGAAAUAGCAA*G*U*U*A*A*AAUAAGGCUAG*U CC*G*U*U*A*UC*A*ACUUGAAAAAGUGGCACCG AGUCGGUGC |
| SEQ ID NO. 22 | Guide-56 | NNNNNNNNNNNNNNNNNNNNNGU*U*U*UAGAGCU AGAAAUAGCAAG*U*U*AAAAUAAGGCUAGUCCG *U*U*A*UCAACUUGAAAAAGUGGCACCGAGUCG GUGC |
| SEQ ID NO. 23 | Guide-63 | NNNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 24 | Guide-67 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 25 | Guide-68 | N*N*N*NNNNNNNNNNNNNNNNNGUUUUAGAGCU AGAAAUAGCAAGUUAAAAUAAGGCUAGUCCGUU AUCAACUUGAAAAAGUGGCACCGAGUCGG*U*G* C |
| SEQ ID NO. 26 | Guide-69 | N*N*N*NNNNNNNNNNNNNNNNNGUUUUAGAGCU AGAAAUAGCAAGUUAAAAUAAGGCUAGUCCGUU AUCAACUUGAAAAAGUGGCACCGAGUCGG*U*G* C |
| SEQ ID NO. 27 | Guide-70 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 28 | Guide-22 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 29 | Guide-23 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 30 | Guide-24 | NNNNNNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |
| SEQ ID NO. 31 | Guide-25 | NNNNNNNNNNNNNNNNNNNNGUUUUAGAGCUAG AAAUAGCAAGUUAAAAUAAGGCUAGUCCGUUAU CAACUUGAAAAAGUGGCACCGAGUCGGUGC |

Figure 1B shows the GFP knockout efficacy of modified guide RNAs versus an unmodified guide RNA targeting the GFP gene stably integrated into the HeLa cell genome. Efficient CRISPR activity results in GFP gene disruption and loss of GFP fluoresence measured by flow cytometry. Modified guide RNAs 1, 2, 17, 37, 38, 39, 41, 42, and 43 are equally efficient or better in reducing GFP expression as shown by percentage of measured GFP-negative cells. The superiority of Guide-1 versus the unmodified guide RNA targeting GFP was further demonstrated by titration experiments (Figure 3A and 3B) showing increased CRISPR activity towards the GFP gene espiacially at low guide RNA concentrations. The need for increased nucleotide modification was also demonstrated by direct comparison of Guide-1 versus Guide-2 as shown by increased CRISPR activity of Guide-1 at low guide RNA concentrations.

### Example 3 - Measurement of cytokine release by modified Guides in mammalian cells

Cytokine release was measured using human peripheral blood mononuclear cells (PBMC). PBMC (Allcells, USA) were transfected with indicated concentrations of guide RNA and 3 µg/ml DOTAP (Roche, CH). After 24h, supernatant was removed and processed using the CBA Human Chemokine Kit (BD, USA) according to manufacturer's protocol and analysed for chemokine intensity using flow cytometry (LSRFortessa, BD Bioscences, USA). See **Figure 5** for results.

Transfection of human peripheral blood mononuclear cells with immune stimulatory RNAs leads to the release of proinflammatory cytokines (here shown by Rantes/CCL5 release as an example). Modifications of guide RNAs prevent the cytokine release after transfection of high concentrations of guide RNAs in comparison to an unmodified guide RNA.

### Example 4 - Comparison with unmodified and 2'O-methyl modified guides

Unmodified and "Guide-1" -like 2'-O-methyl-modified guide RNAs specific for PCSK9 and TTR were tested and compared in vitro using Hepa1-6 cells and primary murine hepatocytes.

Further studies were conducted in vivo using Silence Therapeutics' proprietary liver-specific delivery tools.

### Example 5 - Comparison with unmodified and 2'Fluoro modified guides

In addition, further modifications (e.g. 2'-fluoro) will be tested according to the "Guide-1" pattern.

HeLa cells stably expressing the green fluorescent protein (GFP) will be seeded on 6-well plates (250,000 cells/well) 24h before transfection.

Cells will be co-transfected with Cas9 mRNA and differently 2'-Fluoro-modified guide RNAs targeting the GFP gene.

Transfections will be carried out using Lipofectamine MessengerMax (Life technologies, USA) according to the manufacturer's protocol. After 24h, cells will be trypsinized and transferred to 100 mm cell culture dishes. After 96h cells will be trypsinized, washed twice with PBS and analysed for GFP fluorescence using flow cytometry (LSRFortessa, BD Bioscences, USA).

### Example 6 - Modified guide RNAs for Staphylococcus aureus and Staphylococcus haemolyticus

Natural Staphylococcus aureus guide RNA (SEQ ID NO.32)
NNNNNNNNNNNNNNNNNNNNNGUUUUAGUACUCUGGAAACAGAAUCUACUAAAACAAGGCAAA
AUGCCGUGUUUAUCUCGUCAACUUGUUGGCGAGAU

Guide-100 modification pattern (SEQ ID NO.33)
NNNNNNNNNNNNNNNNNNNNGUUUUAGUACUCUGGAAACAGAAUCUACUAAAACAAGGCAAA
AUGCCGUGUUUAUCUCGUCAACUUGUUGGCGAGAU

Guide-101 modification pattern (SEQ ID NO.34)
NNNNNNNNNNNNNNNNNNNNGUUUUAGUACUCUGGAAACAGAAUCUACUAAAACAAGGCAAA
AUGCCGUGUUUAUCUCGUCAACUUGUUGGCGAGAU

Guide-102 modification pattern (SEQ ID NO.35)
NNNNNNNNNNNNNNNNNNNNGUUUUAGUACUCUGGAAACAGAAUCUACUAAAACAAGGCAAA
AUGCCGUGUUUAUCUCGUCAACUUGUUGGCGAGAU

Guide-103 modification pattern (SEQ ID NO.36)
NNNNNNNNNNNNNNNNNNNNGUUUAGUACUCUGGAAACAGAAUCUACUAAAACAAGGCAAA
AUGCCGUGUUUAUCUCGUCAACUUGUUGGCGAGAU

Guide-104 modification pattern (SEQ ID NO.37)
NNNNNNNNNNNNNNNNNNNNGUUUUAGUACUCUGGAAACAGAAUCUACUAAAACAAGGCAAA
AUGCCGUGUUUAUCUCGUCAACUUGUUGGCGAGAU

Guide-105 modification pattern (SEQ ID NO.38)
NNNNNNNNNNNNNNNNNNNNGUUUAGUACUCUGGAAACAGAAUCUACUAAAACAAGGCAAA
AUGCCGUGUUUAUCUCGUCAACUUGUUGGCGAGAU

Guide-106 modification pattern (SEQ ID NO.39)
NNNNNNNNNNNNNNNNNNNNNGUUUUAGUACUCUGGAAACAGAAUCUACUAAAACAAGGCAAA
AUGCCGUGUUUAUCUCGUCAACUUGUUGGCGAGAU

Guide-107 modification pattern (SEQ ID NO.40)
NNNNNNNNNNNNNNNNNNNNGUUUUAGUACUCUGGAAACAGAAUCUACUAAAACAAGGCAAA
AUGCCCUGUUUAUCUCGUCAACUUGUUGGCGAGAU

Guide-108 modification pattern (SEQ ID NO.41)
NNNNNNNNNNNNNNNNNNNNGUUUAGUACUCUGGAAACAGAAUCUACUAAAACAAGGCAAA
AUGCCGUGUUUAUCUCGUCAACUUGUUGGCGAGAU

Guide-109 modification pattern (SEQ ID NO.42)
NNNNNNNNNNNNNNNNNNNNGUUUUAGUACUCUGGAAACAGAAUCUACUAAAACAAGGCAAA
AUGCCGUGUUUAUCUCGUCAACUUGUUGGCGAGAU

Guide-110 modification pattern (SEQ ID NO.43)

N*N*N*NNNNNNNNNNNNNNNNNGUUUUAGUACUCUGGAAACAGAAUCUACUAAAACAAGGC
AAAAUGCCGUGUUUAUCUCGUCAACUUGUUGGCGA*G*A*U

Guide-111 modification pattern (SEQ ID NO.44)
N*N*N*NNNNNNNNNNNNNNNNNNGUUUUAGUACUCUGGAAACAGAAUCUACUAAAACAAGGC
AAAAUGCCGUGUUUAUCUCGUCAACUUGUUGGCGA*G*A*U

Guide-112 modification pattern (SEQ ID NO.45)
N*N*N*NNNNNNNNNNNNNNNNNNGUUUUAGUACUCUGGAAACAGAAUCUACUAAAACAAGGC
AAAAUGCCGUGUUUAUCUCGUCAACUUGUUGGCGA*G*A*U

Guide-113 modification pattern (SEQ ID NO.46)
N*N*N*NNNNNNNNNNNNNCNNNNGUUUUAGUACUCUGGAAACAGAAUCUACUAAAACAAGGCA
AAAUGCCGUGUUUAUCUCGUCAACUUGUUGGCGA*G*A*U

Guide-114 modification pattern (SEQ ID NO.47)
N*N*N*NNNNNNNNNNNNNNNNNNGUUUUAGUACUCUGGAAACAGAAUCUACUAAAACAAGGC
AAAAUGCCGUGUUUAUCUCGUCAACUUGUUGGCGA*G*A*U

Guide-115 modification pattern (SEQ ID NO.48)
N*N*N*NNNNNNNNNNNNNNNNNNGUUUUAGUACUCUGGAAACAGAAUCUACUAAAACAAGGC
AAAAUGCCGUGUUUAUCUCGUCAACUUGUUGGCGA*G*A*U

Guide-116 modification pattern (SEQ ID NO.49)
N*N*N*NNNNNNNNNNNNNNNNNGUUUUAGUACUCUGGAAACAGAAUCUACUAAAACAAGGC
AAAAUGCCGUGUUUAUCUCGUCAACUUGUUGGCGA*G*A*U

Guide-117 modification pattern (SEQ ID NO.50)
N*N*N*NNNNNNNNNNNNNNNNNNNGUUUUAGUACUCUGGAAACAGAAUCUACUAAAACAAGGC
AAAAUGCCCUGUUUAUCUCGUCAACUUGUUGGCGA*G*A*U

Guide-118 modification pattern (SEQ ID NO.51)
N*N*N*NNNNNNNNNNNNNNNNNNGUUUUAGUACUCUGGAAACAGAAUCUACUAAAACAAGGC
AAAAUGCCGUGUUUAUCUCGUCAACUUGUUGGCGA*G*A*U

Guide-119 modification pattern (SEQ ID NO.52)
N*N*N*NNNNNNUNNNNNNNNNNNGUUUUAGUACUCUGGAAACAGAAUCUACUAAAACAAGGC
AAAAUGCCGUGUUUAUCUCGUCAACUUGUUGGCGA*G*A*U

Guide-120 modification pattern (SEQ ID NO.53)
N*N*N*N*N*NNNNNNNNNN*N*N*NNN*NGU*UUUAGUAC*UCUGGAAACAGAAUCUAC*UAAAA
CA*AGGCA*AAAUGCCG*UGUUUAUCUCGUCAACUUGUUGGCGA*G*A*U

Guide-121 modification pattern (SEQ ID NO.54)
N*N*N*N*N*NNNNUNNNNN*N*N*NNN*NGUUUUAGUAC*UCUGGAAACAGAAUCUAC*UAAAAC
A*AGGCA*AAA*UGCCG*UGUUUAUCUCGUCAACUUGUUGGCGA*G*A*U

Guide-122 modification pattern (SEQ ID NO.55)
N*N*N*N*N*NNNNNNNNNN*N*N*NNN*NGU*UUUAGUACUCUGGAAACAGAAUCUAC*UAAAAC
A*AGGCA*AAA*UGCCG*UGUUUAUCUCGUCAACUUGUUGGCGA*G*A*U

Guide-123 modification pattern (SEQ ID NO.56)

N*N*N*N*N*NNNNNNNNNN*N*N*NNN*NGU*UUUAGUAC*UCUGGAAACAGAAUCUACUAAAAC
A*AGGCA*AAA*UGCCG*UGUUUAUCUCGUCAACUUGUUGGCGA*G*A*U

Guide-124 modification pattern (SEQ ID NO.57)
N*N*N*N*N*NNNNNNNNNN*N*N*NNN*NGU*UUUAGUAC*UCUGGAAACAGAAUCUAC*UAAAA
CAAGGCA*AAA*UGCCG*UGUUUAUCUCGUCAACUUGUUGGCGA*G*A*U

Guide-125 modification pattern (SEQ ID NO.58)
N*N*N*N*N*NNNNNNNNNN*N*N*NNN*NGU*UUUAGUAC*UCUGGAAACAGAAUCUAC*UAAAA
CA*AGGCAAAA*UGCCG*UGUUUAUCUCGUCAACUUGUUGGCGA*G*A*U

Guide-126 modification pattern (SEQ ID NO.59)
N*N*N*NNNNNNNNNNNNN*N*N*NNN*NGU*UUUAGUAC*UCUGGAAACAGAAUCUAC*UAAAACA
*AGGCA*AAAUGCCG*UGUUUAUCUCGUCAACUUGUUGGCGA*G*A*U

Guide-127 modification pattern (SEQ ID NO.60)
N*N*N*N*N*NNNNNNNNNN*N*N*NNN*NGU*UUUAGUAC*UCUGGAAACAGAAUCUAC*UAAAA
CA*AGGCA*AAA*UGCCCUGUUUAUCUCGUCAACUUGUUGGCGA*G*A*U

Guide-128 modification pattern (SEQ ID NO.61)
N*N*N*NN*NNNNNNNNNN*N*N*NNN*NGU*UUUAGUAC*UCUGGAAACAGAAUCUAC*UAAAAC
A*AGGCA*AAA*UGCCG*UGUUUAUCUCGUCAACUUGUUGGCGA*G*A*U

Guide-129 modification pattern (SEQ ID NO.62)
N*N*N*N*NNNNNNNNNNN*N*N*NNN*NGU*UUUAGUAC*UCUGGAAACAGAAUCUAC*UAAAAC
A*AGGCA*AAA*UGCCG*UGUUUAUCUCGUCAACUUGUUGGCGA*G*A*U

### Example 7 - Modified guide RNAs for Cpf1 from Lachnospiraceae bacterium ND2006

Natural guide RNA **(SEQ ID NO.63)**
   UAAUUUCUACUCUAAGUAGAUNNNNNNNNNNNNNNNNNNNN
Guide-201 modification pattern **(SEQ ID NO.64)**
   UAAUUUCUACUCUAAGUAGAUNNNNNNNNNNNNNNNNNNNN
Guide-202 modification pattern **(SEQ ID NO.65)**
   UAAUUGGGGGUCUUCCCCCUNNNNNNNNNNNNNNNNNNNN
Guide-203 modification pattern **(SEQ ID NO.66)**
   UAAUUGCGCGUCUUCGCGCUNNNNNNNNNNNNNNNNNNNN
Guide-204 modification pattern **(SEQ ID NO**.**67**)
   UAAUUUCUACUGUAGAUNNNNNNNNNNNNNNNNNNNN
Guide-205 modification pattern **(SEQ ID NO.68)**
   AAUUUCUACUCUUGUAGAUNNNNNNNNNNNNNNNNNNNN
Guide-206 modification pattern **(SEQ ID NO**.**69)**
   AUUUCUACUCUUGUAGAUNNNNNNNNNNNNNNNNNNNN
Guide-207 modification pattern **(SEQ ID NO.70)**
   UUUCUACUCUUGUAGAUNNNNNNNNNNNNNNNNNNNN
Guide-208 modification pattern **(SEQ ID NO.71)**
   UUUCUACUGUAGAUNNNNNNNNNNNNNNNNNNNN
Guide-209 modification pattern **(SEQ ID NO.72)**
   UAAUUUCUACUCUUGUAGAUNNNNNNNNNNNNNNNNNNNN
Guide-210 modification pattern **(SEQ ID NO.73)**
   UAAUUUCUACUCUUGUAGAUNNNNNNNNNNNNNNNNNNNN
Guide-211 modification pattern **(SEQ ID NO.74)**
   UAAUUUCUACUCUUGUAGAUNNNNNNNNNNNNNNNNNNNN
Guide-212 modification pattern **(SEQ ID NO.75)**
   UAAUUUCUACUCUUGUAGAUNNNNNNNNNNNNNNNNNNNN
Guide-213 modification pattern **(SEQ ID NO.76)**
   U*A*A*UUUCUACUCUUGUAGAUNNNNNNNNNNNNNN*N*N*N
Guide-214 modification pattern **(SEQ ID NO.77)**
   U*A*A*UUGGGGGUCUUCCCCCUNNNNNNNNNNNNNNNN*N*N*N
Guide-215 modification pattern **(SEQ ID NO.78)**
   U*A*A*UUGCGCGUCUUCGCGCUNNNNNNNNNNNNNNNNN*N*N*N
Guide-216 modification pattern **(SEQ ID NO.79)**
   U*A*A*UUUCUACUGUAGAUNNNNNNNNNNNNNNNN*N*N*N
Guide-217 modification pattern **(SEQ ID NO.80)**
   A*A*U*UUCUACUCUUGUAGAUNNNNNNNNNNNNNNNNN*N*N*N
Guide-218 modification pattern **(SEQ ID NO.81)**
   A*U*U*UCUACUCUUGUAGAUNNNNNNNNNNNNNNNNN*N*N*N
Guide-219 modification pattern **(SEQ ID NO.82)**
   U*U*U*CUACUCUUGUAGAUNNNNNNNNNNNNNNNNN*N*N*N
Guide-220 modification pattern **(SEQ ID NO.83)**
   U*U*U*CUACUGUAGAUNNNNNNNNNNNNNNNNN*N*N*N
Guide-221 modification pattern **(SEQ ID NO.84)**
   U*A*A*UUUCUACUCUUGUAGAUNNNNNNNNNNNNNNNNN*N*N*N
Guide-222 modification pattern **(SEQ ID NO.85)**
   U*A*A*UUUCUACUCUUGUAGAUNNNNNNNNNNNNNNNNN*N*N*N
Guide-223 modification pattern **(SEQ ID NO.86)**
   U*A*A*UUUCUACUCUUGUAGAUNNNNNNNNNNNNNNNNN*N*N*N
Guide-223 modification pattern **(SEQ ID NO.87)**
   U*A*A*UUUCUACUCUUGUAGAUNNNNNNNNNNNNNNNNN*N*N*N
Guide-224 modification pattern **(SEQ ID NO.88)**
   U*A*A*UUUCU*ACUC*UUGUAGAU*NNNNNNNNNNNNNNNNN*N*N*N
Guide-225 modification pattern **(SEQ ID NO.89)**
   U*A*A*U*UGGGGGUC*UUCCCCCUN*NNNNNNNNNNNNN*NNN*N*N*N
Guide-226 modification pattern **(SEQ ID NO.90)**
   U*A*A*UUGCGCGUC*UUCGCGCUNNNNNN*NN*NNNNNNNNN*N*N*N
Guide-227 modification pattern **(SEQ ID NO.91)**
   U*A*A*U*UUCUACUGUAGAUNNNNNNNNNNN*NNNN*NN*N*N*N
Guide-228 modification pattern **(SEQ ID NO.92)**
   A*A*U*UUCUACU*CUUGUAGAU*NNNNNNNNNN*NNNNNNN*N*N*N
Guide-229 modification pattern **(SEQ ID NO.93)**
   A*U*U*UCUACU*CU*UGUAGAUNNNNNNNNN*NN*NNNNNN*N*N*N
Guide-230 modification pattern **(SEQ ID NO.94)**
   U*U*U*CUACU*CU*UGUAGAUN*NNNNNNNNNN*NNN*NNN*N*N*N
Guide-231 modification pattern **(SEQ ID NO.95)**
   U*U*U*CUACU*GUAGAUNNNNNNNNNNNNN*N*NNN*N*N*N
Guide-232 modification pattern **(SEQ ID NO.96)**
   U*A*A*U*UUCUACU*C*UUGUAGAUNNNNN*NN*NNNNNNNNNN*N*N*N
Guide-233 modification pattern **(SEQ ID NO.97)**
   U*A*A*U*UUCUAC*U*C*UUGUAGAUNNNNNNNNNNNNNNNNN*N*N*N
Guide-234 modification pattern **(SEQ ID NO.98)**
   U*A*A*UUUCUACUCUUGU*A*G*AUNNNNNNNNNNNNNNNNN*N*N*N
Guide-235 modification pattern **(SEQ ID NO.99)**
   U*A*A*UU*UCUACU*C*U*UGUAGAU*NNNNNNNNNNNNNN*N*N*N

### Example 8 - Modified guide RNAs for Cpf1 from Acidaminococcus sp. BV3L6

Natural guide RNA **(SEQ ID NO.100)**
   UAAUUUCUACUCUUGUAGAUNNNNNNNNNNNNNNNNNNNN
Guide-201 modification pattern (SEQ ID NO.101)
   UAAUUUCUACUCUUGUAGAUNNNNNNNNNNNNNNNNNNNN
Guide-202 modification pattern **(SEQ ID NO.101)**
   UAAUUGGGGGUCUUCCCCCUNNNNNNNNNNNNNNNNNNNN
Guide-203 modification pattern **(SEQ ID NO.102)**
   UAAUUGCGCGUCUUCGCGCUNNNNNNNNNNNNNNNNNNNN
Guide-204 modification pattern **(SEQ ID NO.103)**
   UAAUUUCUACUGUAGAUNNNNNNNNNNNNNNNNNNNN
Guide-205 modification pattern **(SEQ ID NO.104)**
   AAUUUCUACUCUUGUAGAUNNNNNNNNNNNNNNNNNNNN
Guide-206 modification pattern **(SEQ ID NO.105)**
   AUUUCUACUCUUGUAGAUNNNNNNNNNNNNNNNNNNNN
Guide-207 modification pattern **(SEQ ID NO.106)**
   UUUCUACUCUUGUAGAUNNNNNNNNNNNNNNNNNNNN
Guide-208 modification pattern **(SEQ ID NO.107)**
   UUUCUACUGUAGAUNNNNNNNNNNNNNNNNNNNN
Guide-209 modification pattern **(SEQ ID NO.108)**
   UAAUUUCUACUCUUGUAGAUNNNNNNNNNNNNNNNNNNNN
Guide-210 modification pattern **(SEQ ID NO.109)**
   UAAUUUCUACUCUUGUAGAUNNNNNNNNNNNNNNNNNNNN
Guide-211 modification pattern **(SEQ ID NO.110)**
   UAAUUUCUACUCUUGUAGAUNNNNNNNNNNNNNNNNNNNN
Guide-212 modification pattern **(SEQ ID NO.111)**
   UAAUUUCUACUCUUGUAGAUNNNNNNNNNNNNNNNNNNNN
Guide-213 modification pattern **(SEQ ID NO.112)**
   U*A*A*UUUCUACUCUUGUAGAUNNNNNNNNNNNNNN*N*N*N
Guide-214 modification pattern **(SEQ ID NO.113)**
   U*A*A*UUGGGGGUCUUCCCCCUNNNNNNNNNNNNNNNNN*N*N*N
Guide-215 modification pattern **(SEQ ID NO.114)**
   U*A*A*UUGCGCGUCUUCGCGCUNNNNNNNNNNNNNNNNN*N*N*N
Guide-216 modification pattern **(SEQ ID NO.115)**
   U*A*A*UUUCUACUGUAGAUNNNNNNNNNNNNNNNNN*N*N*N
Guide-217 modification pattern **(SEQ ID NO.116)**
   A*A*U*UUCUACUCUUGUAGAUNNNNNNNNNNNNNNNNN*N*N*N
Guide-218 modification pattern **(SEQ ID N0.117)**
   A*U*U*UCUACUCUUGUAGAUNNNNNNNNNNNNNNNNN*N*N*N
Guide-219 modification pattern **(SEQ ID NO.118)**
   U*U*U*CUACUCUUGUAGAUNNNNNNNNNNNNNNNNN*N*N*N
Guide-220 modification pattern **(SEQ ID NO.119)**
   U*U*U*CUACUGUAGAUNNNNNNNNNNNNNNNNN*N*N*N
Guide-221 modification pattern **(SEQ ID NO.120)**
   U*A*A*UUUCUACUCUUGUAGAUNNNNNNNNNNNNNNNNN*N*N*N
Guide-222 modification pattern **(SEQ ID NO.121)**
   U*A*A*UUUCUACUCUUGUAGAUNNNNNNNNNNNNNNNNN*N*N*N
Guide-223 modification pattern **(SEQ ID NO.122)**
   U*A*A*UUUCUACUCUUGUAGAUNNNNNNNNNNNNNNNNN*N*N*N
Guide-223 modification pattern **(SEQ ID NO.123)**
   U*A*A*UUUCUACUCUUGUAGAUNNNNNNNNNNNNNNNNN*N*N*N
Guide-224 modification pattern **(SEQ ID NO.124)**
   U*A*A*UUUCU*ACUC*UUGUAGAU*NNNNNNNNNNNNNNNNN*N*N*N
Guide-225 modification pattern **(SEQ ID NO.125)**
   U*A*A*U*UGGGGGUC*UUCCCCCUN*NNNNNNNNNNNNN*NNN*N*N*N
Guide-226 modification pattern **(SEQ ID NO.126)**
   U*A*A*UUGCGCGUC*UUCGCGCUNNNNNN*NN*NNNNNNNNN*N*N*N
Guide-227 modification pattern **(SEQ ID NO.127)**
   U*A*A*U*UUCUACUGUAGAUNNNNNNNNNNN*NNNN*NN*N*N*N
Guide-228 modification pattern **(SEQ ID NO.128)**
   A*A*U*UUCUACU*CUUGUAGAU*NNNNNNNNNN*NNNNNNN*N*N*N
Guide-229 modification pattern **(SEQ ID NO.129)**
   A*U*U*UCUACU*CU*UGUAGAUNNNNNNNNN*NN*NNNNNN*N*N*N
Guide-230 modification pattern **(SEQ ID NO.130)**
   U*U*U*CUACU*CU*UGUAGAUN*NNNNNNNNNN*NNN*NNN*N*N*N
Guide-231 modification pattern **(SEQ ID NO.131)**
   U*U*U*CUACU*GUAGAUNNNNNNNNNNNNN*N*NNN*N*N*N
Guide-232 modification pattern **(SEQ ID NO.132)**
   U*A*A*U*UUCUACU*C*UUGUAGAUNNNNN*NN*NNNNNNNNNN*N*N*N
Guide-233 modification pattern **(SEQ ID NO.133)**
   U*A*A*U*UUCUAC*U*C*UUGUAGAUNNNNNNNNNNNNNNNNN*N*N*N
Guide-234 modification pattern **(SEQ ID NO.134)**
   U*A*A*UUUCUACUCUUGU*A*G*AUNNNNNNNNNNNNNNNN*N*N*N
Guide-235 modification pattern **(SEQ ID NO.135)**
   U*A*A*UU*UCUACU*C*U*UGUAGAU*NNNNNNNNNNNNNNNNN*N*N*N

### References:-

Diebold (2008) Adv Drug Deliv Rev. Apr 29;60(7):813-23
Jinek et al. (2012) Science 337: 816-821.
Mali et al., Science. 2013 Feb 15; 339(6121): 823-826
Sampson and Weiss (2014), Bioassays 36(1):34;
Sorrentino (1998) Cell Mol Life Sci.Aug;54(8):785-94
Sternberg and Doudna, (2015) Mol Cell 58(4):568;
Zhang et al. (2014) Hum Mol Genet. 23(R1):R40.

## Claims

1. A modified guide RNA for use with the CRIPR/Cas system wherein the guide RNA is modified by a chemical modification of at least one nucleotide at the 2' position and/or backbone modification of thioates.

2. A modified guide RNA according to claim 1 wherein the nucleotides that are modified are selected from a group of nucleotides which interact with the Cas amino acids in the Cas protein to effect binding of the guide RNA to Cas.

3. A modified guide RNA according to claim 1 or 2 wherein the 2'-OH on the nucleotide is replaced with at least one of H, OR, R, halo, SH, SR, NH2, NHR, N(R)2 or CN, wherein R is C1-C6 alkyl, alkenyl or alkynyl and halo is F, Cl, Br or I.

4. A modified guide RNA according to any preceding claim wherein the modifications are 2'-O-methyl and/or 2'F.

5. A modified guide RNA according to any preceding claim wherein the guide RNA is used with the S.pyogenese CRISPR/Cas9 system.

6. A modified guide RNA sequence according to any preceding claim having the sequence and modifications in any one of SEQ ID No. 1, 2, or 4 to 31.

7. A modified guide RNA according to any preceding claims wherein the modified nucleotides have at least 100%, 99%, 98%, 96%, 95%, 90 %, 85%, 80%, 75%, or 70% correspondence to a target nucleotide selected from a gene or DNA.

8. A modified guide RNA according to any preceding claim for use in medicine.

9. Use of a modified guide RNA according to any preceding claim for altering genes by deleting, substituting, repairing or inserting DNA.

10. A method of altering expression of one or more genes in a cells in vitro comprising introducing into a cell a modified guide RNA according to any one of claims 1 to 5 in a CRISPR-Cas system, wherein the guide RNA targets the gene and the Cas protein cleaves the genomic loci of the DNA molecules encoding the one or more gene products, whereby expression of the one or more gene products is altered

11. A CRISPR-Cas system comprising a Cas protein and one or more modified guide RNAs according to any one of claims 1 to 7, wherein the guide RNA corresponds to a target a gene.

12. A vector comprising a CRISPR-Cas system according to claim 11.

13. A cell comprising a CRISPR-Cas system according to claim 11 or a vector according to claim 12.

14. A pharmaceutical composition comprising a modified guide RNA according to any one of claim 1 to 7, a CRISPR-Cas system according to claim 11, a vector according to claim 12 or a cell according to claim 13, and a pharmaceutically acceptable carrier or excipient.

15. A composition comprising a modified guide RNA according to any one of claim 1 to 7 and at least one delivery means selected from GalNAC, polymers, liposomes, peptides, aptameres, antibodies, viral vectors, folate or transferrin.
